(19) Europäisches Patentamt
European Patent Office
Office européen des brevets

(11) Publication number: 0 140 437
A2

(12) # EUROPEAN PATENT APPLICATION

(21) Application number: 84201466.4

(22) Date of filing: 11.10.84

(51) Int. Cl.⁴: C 07 D 205/04

(30) Priority: 21.10.83 GB 8328253

(43) Date of publication of application: 08.05.85 Bulletin 85/19

(84) Designated Contracting States: AT BE CH DE FR GB IT LI LU NL SE

(71) Applicant: SHELL INTERNATIONALE RESEARCH MAATSCHAPPIJ B.V.
Carel van Bylandtlaan 30
NL-2596 HR Den Haag(NL)

(72) Inventor: Wood, Derek Alexander
76 Sterling Road
Sittingbourne Kent(GB)

(72) Inventor: Mason, Ronald Frank
"Kingswood" Westwell
Ashford Kent(GB)

(74) Representative: Hunter, Keith Roger Ian et al,
4 York Road
London SE1 7NA(GB)

(54) **Substituted azetidine derivatives, process for their preparation, and their use as intermediates.**

(57) Substituted azetidine derivatives of formula

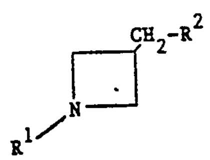

in which $R^1$ represents a methyl group substituted by at least one phenyl group; and $R^2$ represents a chlorine or bromine atom or a hydroxy or alkanoyloxy group of up to 6 carbon atoms, processes for their preparation and their use as intermediates for the preparation of azetidine-3-carboxylic acid derivatives.

EP 0 140 437 A2

0140437

K 1897

# SUBSTITUTED AZETIDINE DERIVATIVES, PROCESS FOR THEIR PREPARATION; AND THEIR USE AS INTERMEDIATES

This invention relates to certain substituted azetidine derivatives which are intermediates for the preparation of biologically active compounds, and to processes for their preparation.

Accordingly the invention provides substituted azetidine derivatives of formula:

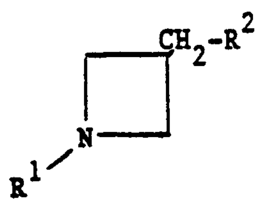

I

in which $R^1$ represents a methyl group substituted by at least one phenyl group; and $R^2$ represents a chlorine or bromine atom or a hydroxy or alkanoyloxy group of up to 6 carbon atoms.

Preferably $R^1$ represents a benzyl group: and $R^2$ represents a chlorine atom or a hydroxy or acetoxy group.

The compounds of formula I in which $R^2$ represents a chlorine or bromine atom may be prepared by a process which comprises reacting a compound of formula:

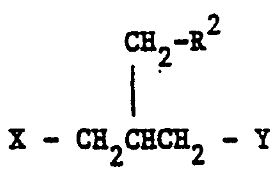

II

in which X and Y each individually represents a chlorine or bromine atom, with a primary amine of formula:

$R^1-NH_2$ III

in the presence of an inorganic base. The inorganic base is preferably an alkali metal hydroxide such as sodium hydroxide. The reaction is suitably carried out in a hydrocarbon solvent such as petroleum spirit. Convenient reaction temperatures are from 50 ° to 100 °C.

The compounds of formula I in which $R^2$ represents an alkanoyloxy group of up to 6 carbon atoms, may be prepared from the corresponding compounds of formula I in which $R^2$ represents a chlorine or bromine atom by reaction with the appropriate alkali metal alkanoate, preferably the sodium alkanoate. The reaction is suitably carried out in a non-aqueous polar solvent such as dimethylformamide. Convenient reaction temperatures are from 100 ° to 150 °C.

The compounds of formula I in which $R^2$ represents a hydroxy group may be prepared from the corresponding compounds of formula I in which $R^2$ represents an alkanoyloxy group of up to 6 carbon atoms by reaction with an alkali metal hydroxide, such as potassium hydroxide. The reaction is preferably carried out in an aqueous alkanol such as aqueous ethanol as solvent.

In each of the above three processes the product may be recovered and purified by conventional procedures.

As mentioned above the substituted azetidine derivatives of the invention are useful intermediates. In particular they may be used as intermediates in a synthetic route to azetidine-3-carboxylic acid derivatives which exhibit plant growth regulant properties especially the property of rendering sterile the male parts of plants. Thus, the compounds of formula I in which $R^2$ represents a hydroxy group may be converted to azetidine-3-carboxylic acid by catalytic oxidation followed by catalytic hydrogenation, using conventional procedures.

Accordingly the invention includes the use of the substituted azetidine derivatives of the invention as intermediates for the preparation of azetidine-3-carboxylic acid derivatives.

The invention is illustrated in the following Examples.

EXAMPLE 1 - 1-Benzyl-3-chloromethylazetidine

(a) Preparation of starting material 2-(Chloromethyl)-3-chloroprop-1-ene (2813 g) containing tert-butyl peroxide (31 g) was stirred at 45-55 °C while gaseous hydrogen bromide (2238 g) was added over a period of 7 hours. The mixture was washed with water and distilled in vacuo to give 1-bromo-2-(chloromethyl)-3-chloropropane as a colourless liquid, b.p. 60-70 °C at 0.2-0.5 mm Hg, in 90 % yield.

(b) Preparation of 1-benzyl-3-chloromethylazetidine 1-Bromo-2-(chloromethyl)-3-chloropropane (103g, prepared as in (a) above) in 100/120 ° petroleum spirit (200 ml) and water (50 ml) was stirred under nitrogen at 80 °C under reflux. Benzylamine (59 g) was added dropwise to the mixture over a period of 2 hours while the pH of the mixture was maintained at 7-7.5. A solution of sodium hydroxide (40 g) in water (60 ml) was then added slowly to the refluxing reaction mixture at a rate sufficient to maintain a pH of about 8. The reaction mixture was cooled and the aqueous phase was removed. The organic phase was washed with water (2 x 100 ml) and the solvent was removed under reduced pressure. The residue was distilled on a wiped-film evaporator to give the desired product as a colourless oil, b.p. 80 °C at 0.01 mm Hg, in 65 % yield.

Analysis

| | |
|---|---|
| Calculated for $C_{11}H_{14}NCl$ | : C 67.5 %; H 7.2 % N 7.2 % |
| Found | : C 67.5 %; H 7.6 % N 6.9 % |

EXAMPLE 2 - 1-Benzyl-3-(acetoxymethyl)azetidine

To a stirred mixture of anhydrous sodium acetate (164 g) and dimethylformamide (1.8 l) at 130 °C was added 1-benzyl-3-chloromethylazetidine (195.5 g, prepared as in Example 1(b)) dropwise over a period of 45 minutes. The reaction mixture was then heated at 130 °C for a further 2½ hours. Most of the solvent was then removed under reduced pressure and the cooled residue was heated with sufficient ice/water mixture to dissolve the salts. The mixture was then extracted with ether and the ether extracts were washed with water (2 x 100 ml). The solvent was then removed from the extracts to yield the required product as a pale yellow oil, b.p. 94 °C at 0.1 mm Hg, in 94 % yield.

Analysis

| | | |
|---|---|---|
| Calculated for $C_{13}H_{17}NO_2$ | : | C 71.2 %; H 7.8 %; N 6.4 % |
| Found | : | C 69.6 %; H 7.9 %; N 6.2 % |

EXAMPLE 3 - 1-Benzyl-3-hydroxymethylazetidine

1-Benzyl-3-(acetoxymethyl)azetidine (191 g, prepared as in Example 2) in ethanol (1.2 l) was heated with potassium hydroxide (65 g, as 85 % aqueous solution). The mixture was allowed to stand at ambient temperature for 16 hours and then solvent was removed under reduced pressure.
The solid residue was dissolved in a small amount of water and extracted with ether (3 x 200 ml). The solvent was removed from the combined extracts under reduced pressure and the residue was distilled on wiped-film evaporator to yield the required product as a pale yellow oil, b.p. 90 °C at 0.02 mm Hg, in 87 % yield

Analysis

| | | |
|---|---|---|
| Calculated for $C_{11}H_{15}NO$ | : | C 74.5 %; H 8.5 %; N 7.9 % |
| Found | | C 73.3 %; H 9.1 %; N 7.9 % |

EXAMPLE 4 - Use of 1-benzyl-3-hydroxymethylazetidine to prepare azetidine-3-carboxylic acid

1-Benzyl-3-hydroxymethylazetidine (17.7 g, prepared as in Example 3) and sodium hydroxide (4.0 g) in water (350 ml) were stirred at 80 °C with a 5 % platinum on charcoal catalyst (7.0 g) while a stream of oxygen was passed into the mixture for two hours. The cooled mixture was then filtered and the water was removed from the filtrate under reduced pressure. The residue was taken up in acetic acid (100 ml) and hydrogenated in the presence of a 5 % palladium on charcoal catalyst (2.4g). The mixture was filtered and the filtrate was evaporated. The solid residue was dissolved in water and passed down a "Dowex" ion exchange column using 2M ammonium hydroxide solution as eluent. Fractions giving a positive ninhydrin test were evaporated to give azetidine-3-carboxylic acid as a white crystalline solid, m.p. 285-290 °C (dec), yield 70 %.

## CLAIMS

1. Substituted azetidine derivatives of formula:

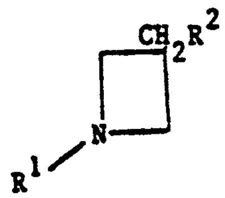

I

in which $R^1$ represents a methyl group substituted by at least one phenyl group; and $R^2$ represents a chlorine or bromine atom or a hydroxy or alkanoyloxy group of up to 6 carbon atoms.

2. Substituted azetidine derivatives as claimed in claim 1, in which $R^1$ represents a benzyl group and $R^2$ represents a chlorine atom or a hydroxy or acetoxy group.

3. A process for the preparation of substituted azetidine derivatives as claimed in claim 1, in which $R^2$ represents a chlorine or bromine atom, which comprises reacting a compound of formula:

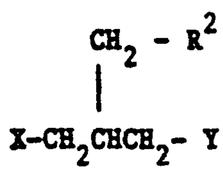

II

in which X and Y each individually respresents a chlorine or bromine atom, with a primary amine of formula:

$$R^1-NH_2 \qquad \text{III}$$

in the presence of an inorganic base.

4. A process for the preparation of substituted azetidine derivatives as claimed in claim 1 in which $R^2$ represents an alkanoyloxy group of up to 6 carbon atoms, which comprises reacting the corresponding compound of formula I in which $R^2$ represents a chlorine or bromine atom with the appropriate alkali metal alkanoate.

5. A process for the preparation of substituted azetidine derivatives as claimed in claim 1, in which $R^2$ represents a hydroxy group, which comprises reacting the corresponding compound of formula I in which $R^2$ represents an alkanoyloxy group of up to 6 carbon atoms, with an alkali metal hydroxide.

6. A process as claimed in claim 3, 4 or 5, substantially as hereinbefore described with reference to Examples 1(b), 2 or 3 respectively.

7. Substituted azetidine derivatives as claimed in claim 1 when prepared by a process as claimed in any one of the claims 3 to 6.

8. The use of substituted azetidine derivatives as claimed in claim 1, 2 or 7 as intermediates for the preparation of azetidine-3-carboxylic acid derivatives.